# EUROPEAN PATENT SPECIFICATION

(11) **EP 3 277 658 B1**
(45) Date of publication and mention of the grant of the patent: **13.02.2019**
(21) Application number: 15808480.6
(22) Date of filing: 30.11.2015
(51) Int. Cl.: C07C 67/11, C07C 41/16, C07C 41/42, C10L 1/02, C11C 3/10, C10G 3/00, C07C 67/03

(54) **METHOD AND SYSTEM FOR THE SYNTHESIS OF BIODIESEL**
VERFAHREN UND SYSTEM ZUR SYNTHESE VON BIODIESEL
PROCÉDÉ ET SYSTÈME DE SYNTHÈSE DE BIODIESEL

(30) Priority: 02.04.2015 PL 41186815
(43) Date of publication of application: 07.02.2018
(73) Proprietor: Biotim Spolka Z Ograniczona Odpowiedzialnoscia, 60-478 Poznan (PL)
(72) Inventor: GASIOREK, Jan, 60-681 Poznan (PL)
(74) Representative: Kancelaria Eupatent.pl Sp. z.o.o
(86) International application number: PCT/PL2015/050063
(87) International publication number: WO 2016/159802

(56) References cited:
- EP-A1- 2 730 567
- WO-A1-2008/111915
- US-A1- 2013 133 245

## Description

### TECHNICAL FIELD

The present invention relates to a method and a system for synthesis of biodiesel comprising methyl esters form plant oils.

### BACKGROUND

Plant oil based fatty acid methyl esters (FAME), popularly known as biodiesel, are utilized as environment-friendly alternative substitutes for diesel fuel.

Biodiesel can be obtained by chemically reacting fatty acids (oil or fat) with alcohol in presence of a homogeneous or heterogeneous catalyst (transestryfiacation reaction). A product of this reaction is a mixture of methyl esters (the biodiesel) and glycerol (which does not have fuel properties).

The process for producing of biodiesel form a biomass by using transestrification reaction is simple and efficient, and it allows to obtain alternative fuel form renewable fuel sources. However, there is a problem in obtaining glycerol, which constitutes transestrification byproduct yielding 20% of the total reaction product, since there is an uncertainty of a secondary market for large volumes of crude glycerol derived from biodiesel manufacture. The low atom economy combined with the glycerol market uncertainty contribute significantly to decrease in profitability of a biodiesel manufacturing plant.

Various patent documents describe the process of manufacture of biodiesel, addressing the problem of utilization of glycerol.

For example, a European patent application EP2730567 describes a method for obtaining simultaneously several compositions comprised of fatty acid alkyl esters (biodiesel), glycerol and fatty acid glycerol bioesters, wherein glycerol is used as a substrate for production of biofuel components. The method for production of the composition consists in reacting triglyceride, glycerol and dialkoxymethane in the presence of an acid catalyst. The reaction product forms two non-mixed layers, where the upper layer comprises fatty acid alkyl esters, fatty acid glycerol esters, alkyl alcohol and excess of dialkoxymethane. The lower layer comprises the glycerol and the catalyst.

A US patent US6712867 describes a process of esterification of triglyceride, which consists in preparing a single phase solution comprising: triglyceride, an alcohol: methanol, a base catalyst and a co-solvent. The solution is mixed at a temperature of 15 to 65°C to cause formation of corresponding methyl esters. The ratio of alcohol to triglyceride is from 15:1 to 35:1. As the co-solvent: etrahydrofuran (THF) and 1,4-dioxane, diethyl ether, methyltertiarybutylether or diisopropyl ether may be used. After the completion of the reaction, the unreacted alcohol and the co-solvent are separated following the separation of the glycerol phase. The remaining layer constitutes the reaction product, i.e. fatty acid methyl esters to be used as a biodiesel.

A European patent application EP2862915 describes a method for production of fatty acid methyl esters form plant oil to be utilized as a biodiesel. The method involves a single stage process consisting in reaction of glycerol with methyl acetate catalyzed by sodium methoxide solution in methanol. The reaction is conducted in a temperature from 40 to 60°C, for 40 to 45 min. To end the reaction, phosphoric acid is added into the mixture of reagents in order to neutralize the catalyst. The reaction products, i.e. acid methyl esters, are next subjected to centrifugation, and optionally cleaning with distilled water.

As follows form the above cited documents, there is a need for further development of a method for synthesis of biodiesel, that will provide utilization of glycerol phase obtained in this process.

### SUMMARY

There present invention relates to a method for synthesis of biodiesel from plant oil and use of a system for synthesis of biodiesel from plant oil accoridng to the appended claims.

### BRIEF DESCRIPTION OF THE DRAWINGS

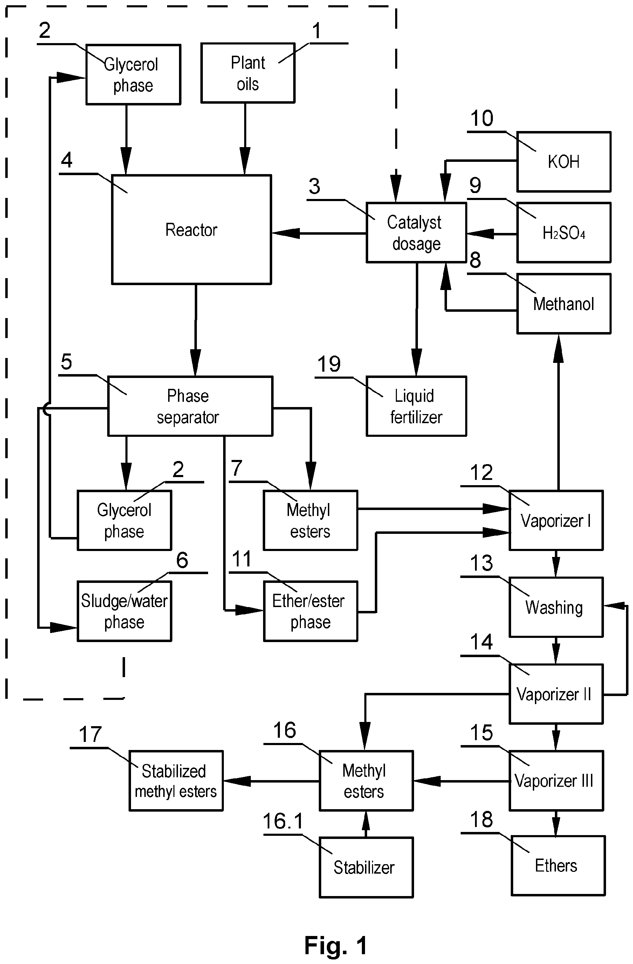

The method is presented by means of exemplary embodiments on a drawing, in which Fig. 1 presents a schematic flowchart of the method for synthesis of biodiesel.

### DETAILED DESCRIPTION

The present invention relates to formation of methyl esters, to be used as biodiesel from plant oils comprising triglycerides.

The method described herein comprises two stages.

The first sage of the process involves the reaction of methanolysis of triglycerides (vegetable oils) with production of methyl esters (biodiesel) and glycerol phase. Thus, the first stage involves the process of transestryfiacation by using methanol and potassium hydroxide as a catalyst.

The second stage of the process involves the reaction of acidolysis of the glycerol phase obtained in the first stage of the process, with production of liquid fertilizer and ether/ester phase that can be used as fuel, thereby providing an efficient method for utilization of glycerol phase constituting the by-product of the transestryfication process.

At the first stage, i.e. the methanolysis (transestryfiction), the oil, e.g. a rapeseed oil or a mixture of different oils, comprising triglycerides, is introduced as a substrate, from an oil dispenser 1 to a reactor 4. The substrate is heated, and when the oil reaches 50°C, the mixture of catalysts comprising methanol and potassium hydroxide is introduced into the reactor 4 to be mixed with the substrate. The mixture of catalysts is prepared in a mixer 3 that may be any suitable tank provided with a stirrer. Methanol and potassium hydroxide are fed from dispensers 8, 10 into the mixer 3. Preferably, 108,4kg of potassium hydroxide (KOH) is used for every 1000 liters of methanol. This preferred methanol:potassium hydroxide ratio is selected due to the stoichiometry of the reaction between these two substances, wherein potassium methoxide is obtained serving as catalyst. Therefore, preferably, for every 1000liters of substrate approximately 178liters of potassium methoxide is utilized.

Preferably, the system comprises comprising at least one vaporizer for distilling methanol and/or water form the polar phase prior to the separation ethers and esters.

### BRIEF DESCRIPTION OF THE DRAWINGS

The method is presented by means of exemplary embodiments on a drawing, in which Fig. 1 presents a schematic flowchart of the method for synthesis of biodiesel.

### DETAILED DESCRIPTION

The present invention relates to formation of methyl esters, to be used as biodiesel from plant oils comprising triglycerides.

The method described herein comprises two stages.

The first sage of the process involves the reaction of methanolysis of triglycerides (vegetable oils) with production of methyl esters (biodiesel) and glycerol phase. Thus, the first stage involves the process of transestryfiacation by using methanol and potassium hydroxide as a catalyst.

The second stage of the process involves the reaction of acidolysis of the glycerol phase obtained in the first stage of the process, with production of liquid fertilizer and ether/ester phase that can be used as fuel, thereby providing an efficient method for utilization of glycerol phase constituting the by-product of the transestryfication process.

At the first stage, i.e. the methanolysis (transestryfiction), the oil, e.g. a rapeseed oil or a mixture of different oils, comprising triglycerides, is introduced as a substrate, from an oil dispenser 1 to a reactor 4. The substrate is heated, and when the oil reaches 50°C, the mixture of catalysts comprising methanol and potassium hydroxide is introduced into the reactor 4 to be mixed with the substrate. The mixture of catalysts is prepared in a mixer 3 that may be any suitable tank provided with a stirrer. Methanol and potassium hydroxide are fed from dispensers 8, 10 into the mixer 3. Preferably, 108,4kg of potassium hydroxide (KOH) is used for every 1000 liters of methanol. This preferred methanol:potassium hydroxide ratio is selected due to the stoichiometry of the reaction between these two substances, wherein potassium methoxide is obtained serving as catalyst. Therefore, preferably, for every 1000liters of substrate approximately 178liters of potassium methoxide is utilized. Preferably, the catalyst is introduced into the reactor 4 to obtain a concentration of the catalyst in the reaction mixture of 12,04%. This allows to obtain appropriate pH of the reaction mixture. The reaction is carried out at the temperature from 50 to 60°C. The pH value is the indicator of the degree of the conversion of the substrates. At the beginning, the pH of the reaction mixture is about 5, and it increases along with the conversion of the substrates. When the reaction mixture reaches pH of 8-9, the reaction is terminated, and the mixture of the reaction products is transferred into a phase separator 5, where the glycerol phase (polar phase) is separated from the phase of methyl esters (non-polar phase). The glycerol phase is collected in the glycerol tank 2 and the phase of methyl esters is collected in a methyl esters tank 7. Next, the collected methyl esters are fed into the system of two vaporizers 12, 14 where the methyl esters are purified by distillation. In the first vaporizer 12, residual methanol is distilled from the mixture of methyl esters, and the methanol vapors are introduced into the methanol dispenser 8, which is connected with a catalyst mixer 3. After the distillation in the first vaporizer 12, the methyl esters are introduced to a washing tank 13 and washed with water in order to remove the impurities of the polar phase (e.g. glycerol). Following the washing process in the washing tank 13, the methyl esters are introduced into the second vaporizer 14 to distill water remains in the mixture of methyl esters after washing. The distilled water is returned to the washing tank 13.

Next, the purified methyl esters are treated with a stabilizer 16.1. The treating may be carried out in a methyl esters tank 16, preferably provided with a stirrer, into which methyl esters are fed preferably directly after distillation in the third vaporizer 15, and are mixed with the stabilizer. A suitable compound useful as a stabilizer is a commercially available Bioxiten 70 (available from Instytut Nafty i Gazu, Poland), comprising: 2-(2-butoxyethoxy)ethanol in the amount of 40 to 44%, 2-(1,1-dimethylethyl)-1,4-benzenediol in the amount of 28 to 32%, 2-(2-methoxyethoxy)ethanol in the amount of 4 to 6%, 2-butoxyethanol in the amount of 1 to 2%, solvent naphtha in the amount of 1 to 2%, (tetrapropenyl)succinic acid in the amount of 1 to 1,5%, and 1,2,4-thrimethylbenzene in the amount of 0,1 to 0,4%. The stabilizer limits the oxidation of the methyl esters and eliminates growth of microorganisms (if present in the esters mixture).

After proper stabilization, the mixture of methyl esters is collected in the stabilized methyl esters tank 17 and may be used as a biodiesel.

The glyceride phase constitutes 20% of the volume of the total product obtained in the reactor 4 (before separation of polar and non-polar phases), thus after each five cycles of the methanolysis the amount of the glycerol phase equals to the volume of the reactor 4. Therefore, after each five cycles of methanolysis, there is provided one cycle of acidolysis of the glycerol phase collected in the glycerol tank 2.

In order to conduct the reaction of acidolysis of glycerol that constitutes the by-product of the process of production of the biodiesel, the glycerol phase is moved from the glycerol tank 2 into the reactor 4. The glycerol phase is heated in the reactor 4, and when the glycerol phase reaches 50°C, the catalyst which constitutes the product of reaction of the methanol and sulphuric (VI) acid is introduced into the reactor 4 to be mixed with the glycerol phase. The catalyst is prepared in the mixer 3. Methanol and sulphuric (VI) acid (H₂SO₄), from dispensers 8, 9, are introduced into the mixer 3. The volume ratio of H₂SO₄:CH₃OH equals 1:1, thus, 1000 liters of methanol are used for 1000liters of sulphuric (VI) acid. This preferred ratio sulphuric (VI) acid /methanol is associated with the stoichiometry of the reaction between this two substances in which dimethyl sulfate in excess sulfuric (VI) acid is obtained serving as catalyst. Preferably, the catalyst is introduced into the reactor 4 to obtain the concentration of the catalyst in the reaction mixture of 7 to 8% v/v. Preferably, 74,5 liters of dimethyl sulfate in excess sulfuric acid are used for 1000 liters of glycerol phase.

This allows to obtain appropriate pH of the reaction mixture. The reaction is carried out within the temperature range of 50 to 55°C, thereby, when the temperature of the reaction mixture exceeds 55°C, the addition of the catalyst is stopped. The pH value is the indicator of the degree of the conversion of glycerol phase. At the beginning, the pH value of the reaction mixture amounts to about 8-9, and it decreases with the conversion of the glycerol phase. When the reaction mixture reaches the pH value of 5-6 due to precipitation of K₂SO₄ whilst the reaction mixture reaches pH value of 1-2 due to precipitation of acid potassium sulfates: KHSO₄., The reaction is terminated at pH value of 5-6 or at pH value 1-2, and the mixture of reaction products, i.e. K₂SO₄ or KHSO₄, is transferred into the phase separator 5.

The mixture of reaction product comprises sludge (polar phase) and ether/ester phase (non-polar phase). The mixture of phases is separated in the phase separator 5. The sludge is collected in the sludge tank 6 and the ether/ester phase is collected in an ether/ester tank 11.

The obtained sludge phase is fed to the mixer 3 where the sludge is neutralized. The neutralization involves treating the sludge with KOH fed into the mixer form the KOH dispenser 10. The neutralized sludge is collected in a liquid fertilizer tank 19. The product of neutralization may be used as a fertilizer, as it contains K₂SO₄ with 29,8% K₂O (% m/m) in dry mass contents.

### Characteristics of the fertilizer product:

| Contents | Unit | Result |
|---|---|---|
| Dry mass | %(m/m) | 96,34 |
| Moisture /H₂O/ | %(m/m) | 3,66 |
| Mineral substances in dry mass | %(m/m) | 58,70 |
| Organic substances in dry mass | %(m/m) | 41,21 |
| Potassium in dry mass | g K/ kg | 247,5 i.e. (29,83%K₂O) |

The solid product characterized in the above table can be used as a potassium fertilizer in agriculture.

The ether/ester phase is fed into the system of three vaporizers 12, 14 and 15 where the ether/ester phase is purified by distillation of methanol and water and then separated so as to separate ethers and esters. At the first vaporizer 12, the residual methanol is distilled from the mixture of ethers and esters, and the methanol vapors are introduced into the methanol volume feeder 8, which is connected with the catalyst mixer 3. After the first vaporizer 12 the ether/ester phase is introduced into the washing tank 13 and washed with water in order to remove polar impurities. Following the washing process in the washing tank 13, the ether/ester phase is introduced into the second vaporizer 14 to distill water remains in the mixture after washing. Water is returned into the washing tank 13. Next, the ether/ester phase is introduced into the third vaporizer 15 to distill the ethers, comprising glycerol methyl ethers, which are collected in an ethers tank 18. The fraction that remains after distillation is a mixture of methyl esters comprising fatty acids methyl esters, which may be treated with a stabilizer 16.1 such as the commercially available Bioxiten 70. Stabilization may be carried out in the methyl esters tank 16 provided with a stirrer. After stabilization, the methyl esters may be collected in the stabilized methyl esters tank 17. The obtained methyl esters may be used as a biodiesel.

The obtained ester or ether/ester blend may be used as a independent fuel suitable for energetic plants or as a biocomponent for use as heating oil.

The invention provides a method for production of methyl esters such as for example: methyl oleate, methyl stearate, methyl palmitate, methyl linoleate, methyl oleate depending on plant oils that have been used as substrates, to be used as a biodiesel. The method provides total elimination of production of glycerol phase. All produced glycerol phase is, according to the present invention, processed into a fertilizer that may be used in agriculture, the ethers fraction to be used as a fuel and methyl esters that can be collected in the tank for methyl esters and used as a biodiesel.

## Claims

1. A method for synthesis of biodiesel from plant oil comprising transestryfication of plant oil with obtaining fatty acid methyl esters phase and glycerol phase, **characterized by** processing the glycerol phase by:
- reacting the glycerol phase in presence of dimethyl sulfate in excess sulfuric (VI) acid as a catalyst within a temperature range of 50 to 55°C to obtain reaction products comprising a polar phase being ether/ester phase and a non-polar phase;
- separating the polar phase form the non-polar phase of the reaction products;
- distilling the polar phase to separate ethers form esters; and
- collecting the ethers and esters in separate tanks.

2. The method according to claim 1, wherein the reaction of glycerol phase with dimethyl sulfate catalyst is carried out to reduce the pH value from 8-9 to the value of 5-6 to obtain neutral potassium sulfate (VI).

3. The method according to claim 1, wherein the reaction of glycerol phase with dimethyl sulfate is carried out to reduce the pH value from 8-9 to the value of 1 - 2 to obtain acid potassium sulfate (VI).

4. The method according to claim 1, wherein dimethyl sulfate constitutes 6 - 8 %v/v of the reaction mixture.

5. The method according to claim 1 or 4, wherein dimethyl sulfate catalyst is obtained by reacting methanol (CH₃OH) with sulphuric (VI) acid (H₂SO₄) with volume ratio of H₂SO₄:CH₃OH amounting to 1:1.

5. The method according to claim 1, wherein ether/ester phase is purified or separated by distillation.

6. The method according to claim 5, wherein separated ester phase is treated with stabilizer.

7. The method according to claim 3, wherein the polar phase containing acid potassium sulfates is mixed with potassium hydroxide (KOH) to obtain a neutral fertilizer comprising potassium sulfate (VI) (K₂SO₄).

8. The method according to claim 1 , wherein the transestryfication of oil plant comprises the steps of:
- reacting the oil plant with potassium methoxide within a temperature range of 50 to 56°C to obtain reaction products comprising a polar phase being methyl ester phase and a non-polar phase;
- separating the polar phase form the non-polar phase of the reaction products; and
- collecting obtained methyl esters in a methyl esters tank.

9. The method according to claim 8, wherein the reaction of plant oil with potassium methoxide is carried out to increase the pH value from 5 to the value of 8-9.

10. The method according to claim 9, wherein potassium methoxide constitutes 12,04%(m/m) of the reaction mixture.

11. The method according to claim 8, wherein the methyl ester phase is treated with stabilizer.

12. Use of a system for synthesis of biodiesel from plant oil by transestryfication of plant oil with obtaining fatty acid methyl esters phase and glycerol phase, wherein the system comprises:
- a reactor (4) for reacting the glycerol phase in presence ofdimethyl sulfate in excess sulfuric acid as a catalyst within a temperature range of 50 to 55°C to obtain reaction products comprising a polar phase being ether/ester phase and a non-polar phase;
- a phase separator (5) for separating the polar phase form the non-polar phase of the reaction products,
- at least one vaporizer (12, 14, 15) for distilling the polar phase to separate ethers and esters; and
- separate tanks (17, 18) for collecting the ethers and esters; for carrying out the method according to any of claims 1-11.

13. Use of the system according to claim 12, wherein the system comprises at least one vaporizer (12, 14) for distilling methanol and/or water form the polar phase prior to the separation ethers and esters.

## Patentansprüche

1. Verfahren zur Synthese von Biodiesel aus Pflanzenöl, umfassend eine Transestrifizierung von Pflanzenöl mit Erlangen von Fettsäuremethylesterphase und Glycerinphase, **gekennzeichnet durch** ein Verarbeiten der Glycerinphase **durch**:
- Reagieren der Glycerinphase in Anwesenheit von Dimethylsulfat in überschüssiger Schwefel(IV)-säure als Katalysator innerhalb eines Temperaturbereichs von 50 bis 55 °C, um Reaktionsprodukte zu erlangen, die eine polare Phase, die Ether/Ester-Phase ist, und eine unpolare Phase umfassen;
- Trennen der polaren Phase von der unpolaren Phase der Reaktionsprodukte;
- Destillieren der polaren Phase, um Ether von Estern zu trennen; und
- Sammeln der Ether und Ester in separaten Behältern.

2. Verfahren nach Anspruch 1, wobei die Reaktion der Glycerinphase mit einem Dimethylsulfatkatalysator durchgeführt wird, um den pH-Wert von 8-9 auf den Wert von 5-6 zu reduzieren, um neutrales Kaliumsulfat (VI) zu erlangen.

3. Verfahren nach Anspruch 1, wobei die Reaktion der Glycerinphase mit Dimethylsulfat durchgeführt wird, um den pH-Wert von 8-9 auf den Wert von 1 - 2 zu reduzieren, um saures Kaliumsulfat (VI) zu erlangen.

4. Verfahren nach Anspruch 1, wobei Dimethylsulfat 6-8 % Vol./Vol. des Reaktionsgemischs ist.

5. Verfahren nach Anspruch 1 oder 4, wobei ein Dimethylsulfatkatalysator durch Reagieren von Methanol (CH₃OH) mit Schwefel(IV)-säure (H₂SO₄) mit einem Volumenverhältnis von H₂SO₄:CH₃OH in Höhe von 1:1 erlangt wird. 5. Verfahren nach Anspruch 1, wobei die Ether/Ester-Phase aufgereinigt oder durch Destillation getrennt wird.

5. Verfahren nach Anspruch 5, wobei die getrennte Esterphase mit einem Stabilisator behandelt wird.

6. Verfahren nach Anspruch 3, wobei die polare Phase, die saure Kaliumsulfate beinhaltet, mit Kaliumhydroxid (KOH) gemischt wird, um einen neutralen Dünger zu erhalten, der Kaliumsulfat (VI) (K₂SO₄) umfasst.

7. Verfahren nach Anspruch 1, wobei die Transestrifizierung von Ölpflanze die folgenden Schritte umfasst:
- Reagieren der Ölpflanze mit Kaliummethoxid innerhalb eines Temperaturbereichs von 50 bis 56 °C, um Reaktionsprodukte zu erlangen, die eine polare Phase, die die Methylesterphase ist, und eine unpolare Phase umfassen;
- Trennen der polaren Phase von der unpolaren Phase der Reaktionsprodukte; und
- Sammeln der erlangten Methylester in einem Methylesterbehälter.

8. Verfahren nach Anspruch 8, wobei die Reaktion von Pflanzenöl mit Kaliummethoxid durchgeführt wird, um den pH-Wert von 5 auf den Wert von 8-9 zu erhöhen.

9. Verfahren nach Anspruch 9, wobei Kaliummethoxid 12,04 % (m/m) des Reaktionsgemischs ist.

10. Verfahren nach Anspruch 8, wobei die Methylesterphase mit einem Stabilisator behandelt wird.

11. Verwendung eines Systems zur Synthese von Biodiesel aus Pflanzenöl durch Transestrifizierung von Pflanzenöl mit Erlangen von Fettsäuremethylesterphase und Glycerinphase, wobei das System Folgendes umfasst:
- einen Reaktor (4) zum Reagieren der Glycerinphase in Anwesenheit von Dimethylsulfat in überschüssiger Schwefelsäure als Katalysator innerhalb eines Temperaturbereichs von 50 bis 55 °C, um Reaktionsprodukte zu erlangen, die eine polare Phase, die Ether/Ester-Phase ist, und eine unpolare Phase umfassen;
- einen Phasentrenner (5) zum Trennen der polaren Phase von der unpolaren Phase der Reaktionsprodukte,
- mindestens einen Verdampfer (12, 14, 15) zum Destillieren der polaren Phase, um Ether und Ester zu trennen; und
- getrennte Behälter (17, 18) zum Sammeln der Ether und Ester; zum Durchführen des Verfahrens nach einem der Ansprüche 1-11.

12. Verwendung des Systems nach Anspruch 12, wobei das System mindestens einen Verdampfer (12, 14) zum Destillieren von Methanol und/oder Wasser aus der polaren Phase vor der Trennung von Ethern und Estern umfasst.

## Revendications

1. Procédé de synthèse de biogazole à partir d'une huile végétale comprenant la transestérification d'huile végétale avec l'obtention d'une phase esters méthyliques d'acides gras et d'une phase glycérol, **caractérisé par** le traitement de la phase glycérol par :
- réaction de la phase glycérol en présence de sulfate de diméthyle dans un excédent d'acide sulfurique (VI) comme catalyseur dans les limites d'une plage de température de 50 à 55 °C pour obtenir des produits de réaction comprenant une phase polaire étant une phase éther/ester et une phase non polaire ;
- séparation de la phase polaire de la phase non polaire des produits de réaction ;
- distillation de la phase polaire pour séparer les éthers des esters ; et
- collecte des éthers et des esters dans des cuves distinctes.

2. Procédé selon la revendication 1, dans lequel la réaction de la phase glycérol avec un catalyseur de sulfate de diméthyle est réalisée pour réduire la valeur de pH de 8 à 9 à la valeur de 5 à 6 pour obtenir du sulfate de potassium (VI) neutre.

3. Procédé selon la revendication 1, dans lequel la réaction de la phase glycérol avec du sulfate de diméthyle est réalisée pour réduire la valeur de pH de 8 à 9 à la valeur de 1 à 2 pour obtenir du sulfate de potassium (VI) acide.

4. Procédé selon la revendication 1, dans lequel le sulfate de diméthyle constitue de 6 à 8 % v/v du mélange de réaction.

5. Procédé selon la revendication 1 ou 4, dans lequel le catalyseur de sulfate de diméthyle est obtenu en faisant réagir du méthanol (CH₃OH) avec de l'acide sulfurique (VI) (H₂SO₄) avec un rapport volumique de H₂SO₄/CH₃OH s'élevant à 1/1. 5. Procédé selon la revendication 1, dans lequel la phase éther/ester est purifiée ou séparée par distillation.

5. Procédé selon la revendication 5, dans lequel la phase ester séparée est traitée avec un stabilisant.

6. Procédé selon la revendication 3, dans lequel la phase polaire contenant des sulfates de potassium acides est mélangée avec de l'hydroxyde de potassium (KOH) pour obtenir un fertilisant neutre comprenant du sulfate de potassium (VI) (K₂SO₄).

7. Procédé selon la revendication 1, dans lequel la transestérification d'une plante oléagineuse comprend les étapes de :
- réaction de la plante oléagineuse avec du méthylate de potassium dans les limites d'une plage de température de 50 à 56 °C pour obtenir des produits de réaction comprenant une phase polaire étant une phase ester méthylique et une phase non polaire ;
- séparation de la phase polaire de la phase non polaire des produits de réaction ; et
- collecte des esters méthyliques obtenus dans une cuve d'esters méthyliques.

8. Procédé selon la revendication 8, dans lequel la réaction de l'huile végétale avec du méthylate de potassium est réalisée pour augmenter la valeur de pH de 5 à la valeur de 8 à 9.

9. Procédé selon la revendication 9, dans lequel le méthylate de potassium constitue 12,04 % (m/m) du mélange réactionnel.

10. Procédé selon la revendication 8, dans lequel la phase ester méthylique est traitée avec un stabilisant.

11. Utilisation d'un système pour la synthèse de biogazole à partir d'une huile végétale par transestérification d'huile végétale avec l'obtention d'une phase esters méthyliques d'acides gras et d'une phase glycérol, dans lequel le système comprend :
- un réacteur (4) pour faire réagir la phase glycérol en présence de sulfate de diméthyle dans un excédent d'acide sulfurique comme catalyseur dans les limites d'une plage de température de 50 à 55 °C pour obtenir des produits de réaction comprenant une phase polaire étant une phase éther/ester et une phase non polaire ;
- un séparateur de phases (5) pour séparer la phase polaire de la phase non polaire des produits de réaction,
- au moins un vaporisateur (12, 14, 15) pour distiller la phase polaire afin de séparer les éthers et les esters ; et
- des cuves distinctes (17, 18) pour collecter les éthers et les esters ; pour réaliser le procédé selon les revendications 1 à 11.

12. Utilisation du système selon la revendication 12, dans lequel le système comprend au moins un vaporisateur (12, 14) pour distiller le méthanol et/ou l'eau de la phase polaire avant la séparation des éthers et des esters.
